# EUROPEAN PATENT APPLICATION

(11) **EP 1 707 167 A1**
(43) Date of publication of application: **04.10.2006**
(21) Application number: 06101062.5
(22) Date of filing: 31.01.2006
(51) Int. Cl.: A61F 13/15

(54) **Procedure to attach belts or side panels to a diaper and system for its realization**

(30) Priority: 02.02.2005 IT PV20050003
(71) Applicant: Viola, Davide, 27010 Valle Salimbene (Pavia) (IT); Viola, Marco, 27010 Linarolo (Pavia) (IT)
(72) Inventor: Viola, Davide, 27010 Valle Salimbene (Pavia) (IT); Viola, Marco, 27010 Linarolo (Pavia) (IT)
(74) Representative: De Gregori, Antonella

(57) **Abstract**

Procedure to attach belts or wings to a diaper and system for its realization where this diaper includes an impermeable external layer, a permeable inside layer, a mediate absorbent pad between the two and two opposite elastic belts fixed to the two sides of the diaper characterized by the fact that these belts are attached to the permeable layer, the joining with all the other elements of diaper happening in continuous succession without never outdistancing him from the conveyer belt (50). The conveyer belt is provided of holes (42) on which the belts are positioned (18) and (20) and the impermeable layer (14), a station of belt's folding, a motor roller (49) to operate the conveyer belt, a series of rollers to carry the various components of the diaper to the conveyer belt, a system to cut and place the belts on the conveyer belt.

## Description

The present invention refers to a procedure to attach diapers belts or wings and system for its realization, and more precisely to a diaper pant.

The diaper pant is made up of a structure with three principal layers such as a permeable inside layer to the liquid that is in contact with the body, an external covering impermeable to the liquid and a mediate absorbent pad between the inside layer and that outside. The materials generally used for the three principal elements of the diapers include various types of nonwoven, a plastic thin sheet for the external covering and pulp of cellulose called fluff for the absorbent pad.

Nonwoven is a comforting material not irritating, proper for the direct contact with the skin and it allows the liquids to filter up to the absorbent pad. The typical absorbent pad is made of pulp of cellulose mixed to polymers super absorbent.

The impermeable external layer is generally a thin plastic sheet that doesn't allow the urines and faeces that are deposited inside the diaper during the normal use to exit to the outside of the same diaper.

These three basic layers that compose the absorbent diaper are generally glued together with hot melt glue, that is distributed in various ways on the inside parts of the various layers and it operates the union of the same in a single product.

Nowadays the absorbent diapers are built with various particularities such us:
two elastic areas on a level with the passage's legs; lateral elastic barriers of containment to prevent the spillage of the liquid and solid substances from the side passage's legs; elastic barriers of containment on a level with the frontal and back part of the diaper to prevent the spillages in the belt's area; various types of elastic bands on a level with the belt to increase the convenience and the wear ability of the diaper; various types of mechanical and elastic side closures by fits and starts or through conventional adhesive tape placed in the back part of the diaper that is possible to replace on the frontal band of the same.

These are some of the most greater characteristics that can be found on the today's absorbent diapers and that they are well known from all the sector's experts. All these novelties are been brought to the absorbent diapers during various years of developments with the purpose to make more and more the comfortable absorbent diapers to wear and proper for to develop their principal assignment to absorb and to contain the liquid and solid substances produced by the body.

The market trend is to use diapers both for children that for adults with two elastic belts on the sides to be able to wear as of the normal diapers but that thanks to the two extensible elastic belts give the comfort of the normal underpants.

To do this it is necessary during the construction of the diaper to insert these two belts to the two sides of the diaper's tape in construction, on a level with the waistline and to refold them inside the same diaper to be able to pack and to group them in bags.

Usually the two side belts are applied on the nonwoven that is found to a superior level of the production's line, 4-8 meters away from the point of assemblage with the rest of the diapers components, refolds, and then insert on-line with the nonwoven.

This system involves however various problems of application such as the difficulty of the belt's positioning control on the nonwoven, the fact that is necessary to build machine on more levels and primarily to collate temporarily the wings on the nonwoven during the transfer of the nonwoven with the belts apply on it to the point of the diaper's forming, where the other components are united for the diaper's forming.

This operation of sticking increases the production's costs.

The technical problem to resolve was to eliminate the present drawbacks in this type of application with the creation of a new application's system for the side belts that it allows a perfect control of the positioning, folding, belt's application and transfer of the belts to the diaper without use temporary fixing systems of the belts themselves.

The solution of the technical problem is characterized for the fact that the belts are attached to the permeable layer, the joining with all the other elements of diaper happening in continuous succession without never outdistancing from the conveyer belt.

Further characteristics and advantages will appear more clearly from the following description and from the enclosed drawings, such us:
the figure 1 is a perspective vision of the diaper object of the present invention;
the figure 2 is a side sight of the device object of the present invention;
the figure 3 is a sight in plant of the device of figure 2.

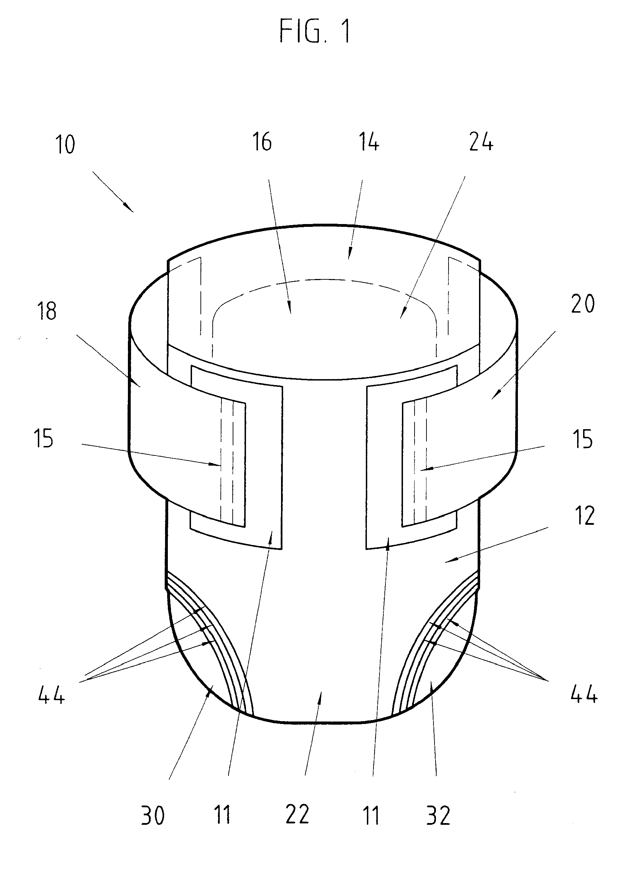

With reference to the figure 1 has been indicated with (10) a diaper pant that includes a body formed by an external layer (12) impermeable, an inside layer (14) permeable, an absorbent pad (16) mediate between the two, and two opposite elastic belts (18) and (20) fixed to the two sides of the diaper between the external layer (12) and the inside layer (14). The two frontal bands (11) are used to fixing the elastic belts (18) and (20) to the diaper's anterior part called in the sector *"Loop".*

The two bands (15) are the elements of anchorage fixed to the wings that allow the reinstatement of the wings called in the sector "Hooks".

The central body once folded up defines a frontal panel (22) and a back panel (24) united by the two opposite elastic belts (18), (20) which define the elastic panels side and they delimit the special opposite openings for the legs (30), (32).

Moreover, a series of elastic members (44) are furnished on the peripheral area of the openings for the legs (30), (32) to substantially form some elastic portions around the openings the legs in the diaper pant (10).

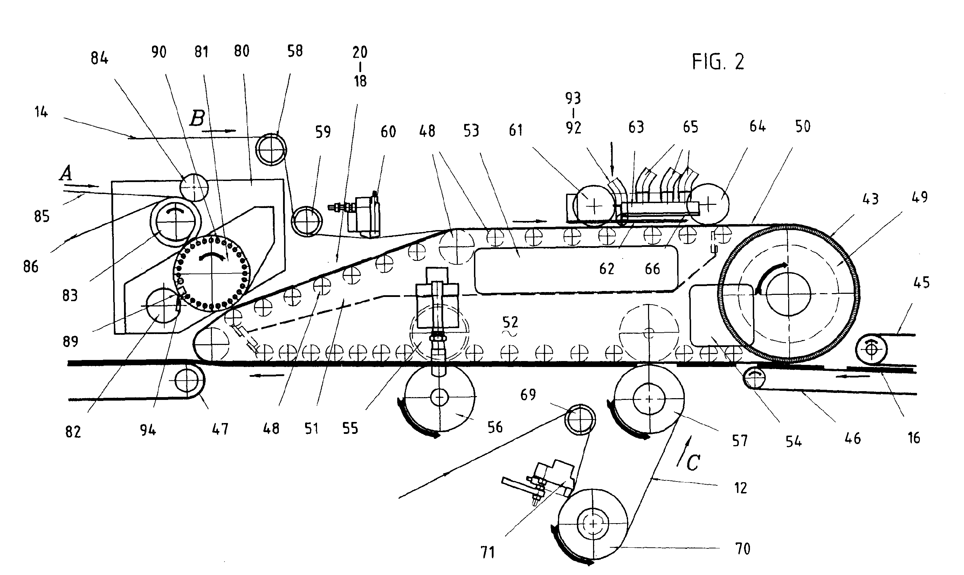

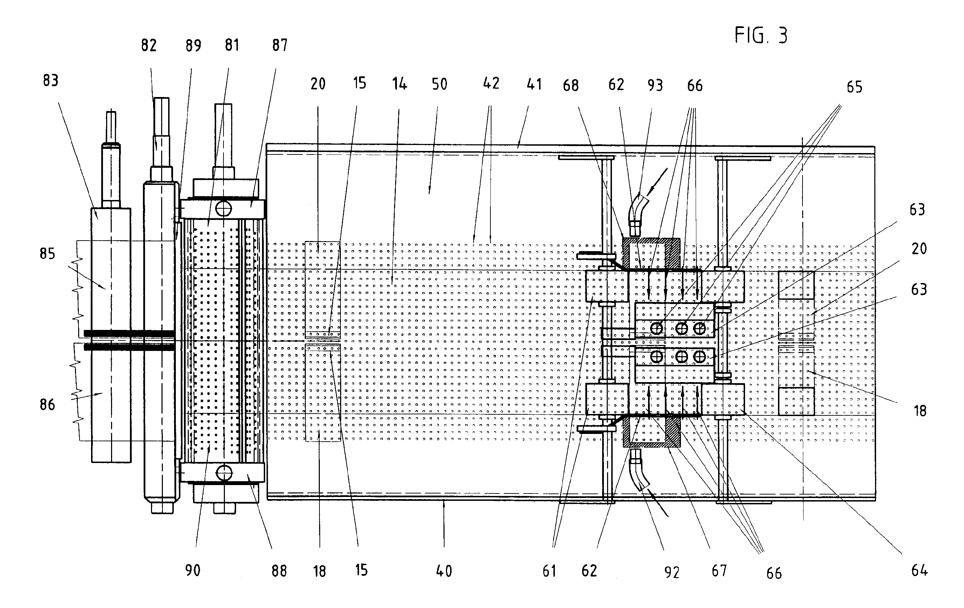

With reference to the figures 2 and 3, an empty conveyer belt (50) is a conveyer belt that through a series of holes (42) on the surface allow to withhold through the depression belts (18) and (20) to apply to the diaper. Inside the conveyer belt delimited by two side walls (40), (41) there are two rooms of depression (51) and (52), the depression is gotten through special non illustrated fans and known in the technique that inhaling air through special openings (53) and (54) in the rooms (51) and (52) they create depression inside the conveyer belt that through the holes (42) on it practised withhold the elements find on it, in our case they are specifically the belts (18) and (20) to apply on the diaper, the nonwoven (14) that it is the diaper's filtering part, the absorbent pulp (16) that forms the diaper's absorbent body, the external plastics (12) that forms the diaper's external impermeable covering.

The conveyer belt (50) has put in movement from a motor roller (49) provided of special holes (43) of air passage to be able to maintain the depression on the external surface of the conveyer belt (50).

The conveyer belt's speed (50) is equal to the speed of the production's line as the conveyer belt is integrated to the production's line.

Inside the conveyer belt (50) are positioned various rollers of support (48) to oppose the effect of the depression that would have the tendency to bend the conveyer belt toward the inside.

There is then a roller with greater diameter (55) that acts as contrast for the pressing of the various diaper's components with the movable motorized rubberised roller (56) placed to the outside of the conveyer belt.

To the outside of the conveyer belt (50), there is the roller (57) which is the roller of insertion of the plastics (12) that will form the impermeable part of the diaper.

The group (80) is the system of cut and place of the belts (18) and (20) on the empty conveyer belt (50).

It is composed from an empty roller (81) in steel on whose external surface are present some holes (90) in which through a special fan and special ducts and cams of aspiration (87), (88) is created a depression only in specific areas, to withhold the bands (85), (86) that will form the belts and the belts themselves (18), (20) that are formed by the cut of bands (85) and (86).

The roller (81) also acts from contrast anvil to the knife roller (82).

The knife roller (82) and a roller on which is applied a cutting blade (89) that turning comes in contact with the anvil (94) climbed on the empty roller (81) and cut in continuous the bands (85), (86) forming so the belts (18), (20).

The rollers (83) and (84) are the rollers that carry the bands (85) and (86) for the forming of the belts and they provide to prepare them on the empty roller (81) for the following cut and place on the empty conveyer belt (50).

The rollers (58) and (59) are rollers for the insertion of nonwoven on the empty conveyer belt (50).

The glue gun (60) is an application head's hot melt glue that serves for the fixing of belts to the nonwoven and of the various diaper's components.

The anti-adhesive rollers (61) are used to maintain stretched the nonwoven before the belts folding inside the nonwoven.

The folding sticks (62) are used for the folding of the two belts inside part.

The aspiration mouths (63) are used for inhaling the belt's inside part to maintain them in tension during the folding.

The anti-adhesive rollers (64) are used for fixing the belt's inside part to the nonwoven one refolded.

The continuous nonwoven (14) will be one assembled the filtering diaper's part. The continuous plastic film (12) will be once assembled the external impermeable diaper's part.

The pulp (16) will be the absorbent central diaper's part that will be inserted among the superior nonwoven (14) and the inferior plastic film (12).

The belts (45) and (46) are conveyer belts of the formed and separate pulp (16) ready to be inserted between the plastic film (12) and the superior nonwoven (14).

The conveyer belt (47) is the continuation of the production's line.

The two bands (85), (86) that will form the elastic belts (18) and (20) are provided in continuous way by own spools (not illustrated) and they move in the direction of job according to the sense of advancement marked in figure 2 from the arrow A, they are driven and maintained to the correct tension of job by special systems (not illustrated) well known in this sector.

In the two bands (85), (86) have already been applied the systems of fixing hook (15) so as that during the insertion in the applicator (80) they come into direct contact with the empty roller (81) and so as that into the finished products they are in contact with the external part of the nonwoven (14).

The bands insertion roller (83) jointly with the contrast roller (84) provide to proceed the bands (85), (86) according to the belt's desired dimension.

The rollers rotation (83), (84) relative to every turn of roller knife (82) correspond to the length of cut of the belts (18), (20) that can be varied according to the various diaper's size.

The empty roller (81) receives the two bands (85), (86) and through the holes (90) and the depression in them created it provides to carry the bands on its surface.

The diameter of the empty roller (81) is considered in such way that the peripheral speed of rotation results smaller in comparison to the peripheral speed of the empty conveyer belt (50) during the manufacture of the diaper, in such way that the passage of the two belts from the empty roller (81) to the empty conveyer belt (50) is always in rising of speed and there is always a factor of draught from the conveyer belt (50).

The knife roller (82) has the function to support the blade (89) that coming in contact during the rotation with the anvil (94) assembled on the roller (81), it provides the cut of the two bands (85), (86) in the belts (18), (20).

The empty roller (81) receives the two bands (85), (86) and through the holes (90) and the depression in them created it provides to carry the bands on his surface.

Since the speed of advancement of the bands related to the roller (81) results to be lower, a continuous slide of the empty roller (81) it will be had in comparison to bands themselves, the blade (89) coming into contact with the anvil posts on the empty roller (81) cut the bands (85), (86) forming so the two belts (18), (20), from the moment of the cut the two belts (18), (20) not being withheld by the bands anymore (85), (86), they take the peripheral speed of the empty roller (81) and they continue with it the rotation always withheld by the holes (90) up to the transfer on the conveyer belt (50).

The cams of aspiration (87) and (88) placed on the sides of the empty roller are conformed in such way by to interrupt the depression in the roller (81) really in match of the tangency of the roller (81) with the conveyer belt (50), so making the belts (18), (20) withheld by the depression not anymore they transfer him from the roller (81) to the conveyer belt (50).

The conveyer belt (50), also it in depression, stirs to the same tangential speed of the nonwoven and it carries the two belts (18), (20) under to the nonwoven (14) already positioning her in their ultimate position, continuing in the advancement of the conveyer belt (50) the two belts (18), (20) positioned under to the nonwoven (14) they bring him to the area of inside folding of the two edges of the belts (18), (20).

As from figure 3 the two belts are positioned on the conveyer belt (50) so that the edge of every belt that will have to be refold on the nonwoven escapes from the same nonwoven.

The nonwoven (14) that will form then the filter part of the diaper is provided in continuous way by own spool (not illustrated) it stirs in the direction of the arrow B of job of the line and it is driven and maintained to the correct tension of job by special systems (not illustrated) well known in this sector. The nonwoven (14), subsequently passes from the rollers (58), (59) and in contact of the glue gun (60) which provides to distribute some lines of glue on the surface of the nonwoven, which will serve both for the fixing of the belts once refolded and for the general fixing of the diaper, continuing his run the nonwoven it brings in contact his side without glue with the conveyer belt (50) and the two belts (18), (20) already deposited on the conveyer belt (50) ready for the folding of the part that escapes from the nonwoven. Two rollers (61) with covering with silicone covering anti-adhesive positioned around two external sides the nonwoven provide to maintain the side edges of the nonwoven in contact with the conveyer belt (50) two nozzles (92), (93) provide through a puff of compressed air to lift the two edges of the belts (18), (20) that they escape from the edges of the nonwoven and to blow them toward the inside where the two empty mouths (63) positioned parallel to the refold edge of the belts receive them, and through the depression they maintain them in tension toward the inside up to their following fixing that happens when the belts by now folded up on the nonwoven (14) and the same nonwoven (14) also pass among the wheels of fixing (64) these last ones you dress again with material of silicon anti-adhesive.

To favour the lifting of the part of belts to fold up two areas (67) and (68) they are created under of not aspiration to the conveyer belt (50) in match of the pixel of lifting of the two edges of the belts to fold up. This way doing when the conveyer belt (50) transport the belts (18), (20) in this area where depression there is not the edges of the belts (18), (20) they are released by the conveyer belt (50) and they can bend inclined from the puff of air of the nozzles (92),(93).

On the empty mouths (63) the depression is gotten through a fan (not in figure) that inhaling the air through the superior passage (65) creates a stream of air through the crack (66) drawn in the mouthpieces, the stream of air that enters through the opening (66) as pointed out by the arrows, it maintains the edge of the belts in tension during the process of folding.

To perform belt's folding (18), (20) it also uses two contrast sticks (62) around which the belts are spooled during the folding, the two sticks (62) are positioned along the two sides of the nonwoven between the wheels (61) and (64) and they have the function to oppose the strength of aspiration of the mouths (63), and to maintain the position of the parallel folding to the sides of the nonwoven (14).

The conveyer belt (50) continues on its movement and brings the nonwoven (14) and the belts (18), (20) to it applied and already refolded to the following joining with the component remainders the diaper.

The absorbent pad (16) already cut and outdistanced are brought in tangency with the conveyer belt (50) to pair with the nonwoven (14) and the belts (18), (20) from the two conveyer belts (45) and (46), in the following line the belt's folding (18), (20), the present depression on the conveyer belt (50) is increased thanks to the room (52) so as the absorbent pad (16) and the remaining materials that will compose the diaper perfectly join him with the nonwoven (14). Advancing with the conveyer belt (50) there is the introduction of the plastic film (12), that will form then the diaper's external impermeable part, it is furnished in continuous way by own spool (not illustrated) it move in the direction of the production line, in the direction of the arrow C and it is driven and maintained to the correct tension of job by special systems (not illustrated) well known in this sector, generally on the plastic film during the joining with the rest of the diaper's components the elastic side for the legs have been already applied (44) and the frontal bands loop or similar with already well known operations from the sector's experts.

The plastic film (12) afterwards pass from the rollers (69), (70) and (57) and in contact of the glue gun (71) through which is applied some hot glue on the surface of the film concerned, the hot glue distributed on the plastic film will serve both for the fixing of the belts already refolded and for the general fixing of the whole diaper.

Passing from the roller (57) the plastic film (12) brings him in proximity of the conveyer belt (50) going to pair off with the nonwoven (14) the absorbent pads (16) the parts of refolded belts inside the nonwoven (18), (20) that they are already prepared on the conveyer belt (50).

As the plastic film doesn't allow the air's passage while the nonwoven (14) and the absorbent pad (16) allow it, once that all the materials are paired off, the depression in the conveyer belt (50) it provides to eliminate the present air in the so formed line predisposing it for the following pressing that will happen through, the conveyer belt (50), the rollers (55) and (56).

The roller (55) is a contrast roller placed inside the conveyer belt (50) and it serves to oppose the pressure practiced by the roller (56) of pressing, the roller (56) is a motorized roller that rotates to the same peripheral speed of the conveyer belt (50), and it is covered of rubber, the diaper's line so definitely formed passing through the conveyer belt (50) with the relative contrast roller (55) and the pressing roller (56) is definitely pressed in such a way as to have the adhesion of all the components that compose her.

Continuing in her way the diapers formed line on move in the direction of the conveyer belt of the production continuation (47) that will provide to transport her to the following manufacturing of cut, folding and diaper's package.

The principles, the preferential versions, the ways of operation of the present invention have been described in the above mentioned specifications. The invention doesn't have to be understood as limited to the explained particular forms, because they are understood as indicative and not restrictive.

Moreover, variations and changes can be made by experts in this sector without moving away from the spirit of the invention. The description refers to a procedure and system to attach belts to a diaper pant but the same procedure can be used without changing its concept of application to attach wings or other types of inserts to all the diapers types.

## Claims

1. Procedure to attach belts or wings to a diaper and system for its realization where this diaper includes a body formed by an impermeable external layer, a permeable inside layer, a mediate absorbent pad between the two and two opposite elastic belts fixed to the two sides of the diaper **characterized by** the fact that these belts are attached to this permeable layer, the joining with all the other elements of this diaper happening in continuous succession without never outdistancing it from the conveyer belt.

2. Procedure according to the claim 1 **characterized by** the fact that these belts are positioned among this conveyer belt and this permeable layer.

3. Procedure to attach belts or wings to a diaper and system for its realization **characterized by** the fact that system includes a conveyer belt provided of holes on which these belts and this permeable layer are positioned, a folding station of these belts, a roller motor to operate this conveyer belt, a series of rollers to carry the various components of this diaper to this conveyer belt, means provided to cut and place these belts on this conveyer belt.

4. Procedure according to the claim 3 **characterized by** the fact that these means include a conveyer belt to transport the material of these belts on an empty roller and cooperate with a knife to cut to the desired measure this material, these belts are holded in position on this roller from the depression gotten in this roller before being deposited on this conveyer belt.

5. Procedure according to the claim 4 **characterized by** the fact that the peripheral speed of this roller is equal or inferior to the speed of this conveyer belt.

6. Procedure according to the claim 3 **characterized by** the fact that this station of folding includes some anti-adhesive rollers to maintain extended says permeable layer before the folding of these belts, of the sticks of folding that use for the folding of the inside part of these belts, the aspiration mouths that serve for inhaling the inside part of these belts so that to maintain her in tension during the folding.

7. Procedure according to the claim 3 **characterized by** the fact that it is provided at least a room of depression to withhold these components the diaper on this conveyer belt.

8. Procedure according to one of the preceding claims **characterized by** the fact that it is provided at least a belt to fix to this permeable layer.

9. Procedure to attach belts or wings to a diaper and system for its realization according to as above described and illustrated in the attached drawings.
